# EUROPEAN PATENT APPLICATION

(11) **EP 1 576 968 A2**
(43) Date of publication of application: **21.09.2005**
(21) Application number: 05005774.4
(22) Date of filing: 16.03.2005
(51) Int. Cl.: A61K 49/00

(54) **Dental-caries detecting solution**

(30) Priority: 17.03.2004 JP 2004077092
(71) Applicant: Nippon Shika Yakuhin KK., Yamaguchi, 750-0015 (JP)
(72) Inventor: Ito, Kazuo, Shinagawa-ku, Tokyo, 142-0063 (JP); Oikawa, Misa, Kawasaki-shi, Kanagawa, 211-0041 (JP); Kusunoki, Mizuho, Chiba-shi, Chiba, 263-0016 (JP); Yokota, Kazuyoshi, Shiomonoseki-shi, Yamaguchi, 751-0878 (JP); Tsunekawa, Masayoshi, Shimonoseki-shi, Yamaguchi, 751-0836 (JP)
(74) Representative: Vossius & Partner

(57) **Abstract**

Disclosed is a dental-caries detecting solution obtainable by dissolving a dye capable of staining a carious region of a tooth to provide enhanced visibility of the carious region, in a water-miscible organic solvent, which is capable of distinctively staining only a first decalcified layer, or an outer layer of a softened dentine, to be removed, absolutely without staining a second layer, or an inner layer of the softened dentine, which should not be removed. In the dental-caries detecting solution, the water-miscible organic solvent comprises a compound having a carbon number of 11 or more and including one or more hydroxyl groups. The water-miscible organic solvent is adjusted such that the dental-caries detecting solution has a viscosity of 300 to 1500 Cp.

## Description

The present invention relates to a dental-caries detecting solution for use in treating a carious tooth during a dental care to stain a minimum carious region to be removed for restoring the tooth, and allow the stained carious region to be accurately distinguished from the remaining region in accordance with the difference in hue.

In the treatment for dental caries, a dentist often runs into a situation where a tooth substance has to be removed for restoration.

Dental caries means a symptom in which softening and decay due to bacterial infection occurs in the hard tissue of a tooth, and a deeply invasive/progressive decay is formed in the tooth, which is also simply called "caries".

The researches on factors causing the dental caries have been vigorously made with concerted efforts of the dental field, and bacteria causing the dental caries have already been specified these days.

When this dental caries reaches the enamel-dentine interface and further progresses, the dentine is affected by dental caries. This phenomenon is referred to particularly as "dentine caries", and the resulting carious dentine is referred to as "softened dentine" because it is softened due to bacterial infection.

A treatment for dental caries is completed by fully removing a region affected by dental caries, and restoring the formed cavity using a restorative material.

In the past when a restorative material had poor adhesiveness to a tooth substance, there was no choice but to select a restorative procedure in conformity to the principles of expansion for prevention, the retention of the restorative material and a resistant form. Thus, the amount of tooth substance to be removed was increased beyond necessity, or was inevitably determined in advance, including a part of healthy tooth substance, and the size of dental caries was no more than a secondary factor.

However, at the present day, a restorative material has achieved progress in adhesiveness to a tooth substance. Specifically, in connection with the advent of adhesive resin, a restoration technique has been changed such that a region to be restored and the amount of tooth substance to be removed can be defined by a carious region itself to perform a treatment by removing only a minimum tooth substance. In this treatment, it is important to accurately distinguish a carious region to be removed.

While a carious region to be removed has been empirically determined on the basis of a criterion, such as color and/or hardness of a tooth substance, it has involved a problem about variations in criterion depending on dental operators. Thus, there has been a strong need for a means for informing any dentist about a criterion for a carious region to be removed, with high repeatability.

The following Non-Patent Publications 1 to 6 disclose a series of histological research results on carious dentine. According to these publications, it was found out that a softened dentine consists of two layers of a first decalcified dentine as an outer layer (first layer) and a second decalcified dentine as an inner layer (second layer). It was also verified that, while these first and second layers are commonly a softened dentine, bacterial infection is observed only in the first layer significantly softened, but the second layer semi-decalcified without bacterial infection can be re-calcified by an appropriate treatment.

That is, it is clinically important to remove only the first layer. From this point of view, a dental-caries detecting solution has been developed which is capable of selectively staining only the first layer and visually discriminating a tooth substance to be removed, in accordance with the resulting difference in hue, so as to remove only the first layer. The following Non-Patent Publication 7 discloses a recommended method for minimizing sacrifices in tooth substance by using staining with a dental-caries detecting solution as a marker and removing only a stained region.

However, as to this dental-caries detecting solution, the following subsequent Non-Patent Publication 8 points out that, when a region stained with the dental-caries detecting solution is clinically used as a maker for removing a tooth substance, the entire decalcified layers are undesirably removed, or the second layer cannot be left. Based on the knowledge that even the second layer with no bacterial infection has a pale pink stain with the dental-caries detecting solution, this publication proposes that a region having the pale pink stain should be left without removal.

The following Non-Patent Publication 9 supports this proposal by reason that while the floor of a carious cavity (carious dentine) exhibits gradual staining to be changed from a pale pink stain to no-stain when the dental-caries detecting solution is applied thereto, a region having the pale pink stain should not be subjected to removal because no bacterial infection is observed in this region.

Further, the following subsequent Non-Patent Publication 10 points out that the second layer having no bacterial infection consists of an opaque layer as a surface layer and a transparent layer as a deep layer, and a removal operation performed in accordance with only staining involves the risk of excessively removing the second layer to the extent of reaching the transparent layer or deep layer, because the opaque layer or surface layer is likely to be slightly stained red with the dental-caries detecting solution, and recommends the aforementioned rule that "a region having the pale pink stain should be left".

Heretofore, a dental-caries detecting solution itself has been disclosed in various patent publications.

The following Patent Publication 1 discloses a dental-caries detecting solution containing basic fuchsin, and the following Patent Publication 2 discloses a dental-caries detecting solution prepared by dissolving a given amount of either one of various food or edible dyes in either one of mono-, di- and tri-hydroxy compounds each having a carbon number of 2 to 10, water and compounds thereof, which is currently used in actual clinical practice. The following Patent Publication 3 discloses a dental-caries detecting solution prepared by dissolving a dye in either one of mono-, di- and tri-hydroxy compounds each having a carbon number of 2 to 10, water and liquid mixtures thereof, and the following Patent Publication 4 discloses a dental-caries detecting solution prepared by dissolving basic fuchsin in a liquid of organic mono- or di-hydroxy compound having a carbon number of 2 to 6, and water. The following Patent Publication 5 discloses a halogenated phenyl ether-based antibacterial dental-caries detecting solution comprising a dye and a cationic bactericide. The following Patent Publication 6 discloses an antibacterial dental-caries detecting solution comprising water and/or a water-miscible solvent, a dye, and a polymeric antibacterial compound including a quaternary ammonium base having a (meth) acryloyl or styrene group within a molecule thereof, and the following Patent Publication 7 discloses an antibacterial dental-caries detecting solution comprising water and/or a water-miscible solvent, a dye, and a polymeric antibacterial agent including a (meth) acryloyl or styrene group, and a quaternary-ammonium or pyridinium base, within a molecule thereof.

All of the dental-caries detecting solutions disclosed in these Patent Publications are intended to provide enhanced dye permeability to a carious region so as to clearly stain the carious region, and set forth that a water-miscible organic solvent to be used therein preferably has a viscosity of 20 Cp or less. That is, in each of the dental-caries detecting solutions disclosed in these Patent Publications, a water-miscible organic solvent having a carbon number of 2 to 10 is used to adjust the viscosity of the dental-caries detecting solution to have a relatively low value (20 Cp or less).
[Patent Publication 1] Japanese Patent Laid-Open Publication No. 51-038428
[Patent Publication 2] Japanese Patent Laid-Open Publication No. 55-076821
[Patent Publication 3] Japanese Patent Publication No. 56-048490
[Patent Publication 4] Japanese Patent Publication No. 57-026485
[Patent Publication 5] Japanese Patent Laid-Open Publication No. 10-236914
[Patent Publication 6] Japanese Patent Laid-Open Publication No. 10-236915
[Patent Publication 7] Japanese Patent Laid-Open Publication No. 2000-063290

[Non-Patent Publication 1] The Journal of the Stomatological Society, Japan, 37: p. 279-286, 1970
[Non-Patent Publication 2] The Journal of the Stomatological Society, Japan, 40: p. 65-74, 1973
[Non-Patent Publication 3] The Journal of the Stomatological Society, Japan, 40: p. 306-315, 1973
[Non-Patent Publication 4] The Journal of the Stomatological Society, Japan, 41: p. 202-211, 1974
[Non-Patent Publication 5] The Journal of the Stomatological Society, Japan, 46: p. 269-292, 1979
[Non-Patent Publication 6] The Journal of the Stomatological Society, Japan, 48: p. 104-151, 1981
[Non-Patent Publication 7] The Japanese Journal of Conservative Dentistry, 22: p. 261-264, 1979
[Non-Patent Publication 8] The Journal of the Stomatological Society, Japan, 48: p. 362-385, 1981
[Non-Patent Publication 9] The Journal of the Stomatological Society, Japan, 54: p. 241-270, 1987
[Non-Patent Publication 10] Dental Outlook, 92: p. 996-1000, 1998

However, in the above conventional dental-caries detecting solutions, if only an infected dentine (first layer) to be essentially removed is cut away or removed, a pale-pink-stained region will be left in the floor of a carious cavity (second layer). Thus, when the treated cavity is filled with a semi-transparent resin restorative material, the penetration or permeation of the pink color into the restorative material causes an esthetic problem. If a tooth substances is removed to the extent of reaching the pale-pink-stained region by giving priority to esthetic appearance, it will be excessively removed, or in a preposterous situation.

It is an object of the present invention to provide a dental-caries detecting solution obtainable by dissolving a dye capable of staining a carious region of a tooth to provide enhanced visibility of the carious region, in a water-miscible organic solvent or a mixture of a water-miscible organic solvent and water, which is capable of staining only the first layer clearly and distinctively, absolutely without staining the second layer which should not be removed.

The inventors found that the above object can be achieved by mixing a compound which has a carbon number of 11 or more and includes one or more hydroxyl groups, with a solvent for a dental-caries detecting solution, and adjusting the viscosity of the dental-caries detecting solution in the range of 300 to 1500 Cp. Based on this knowledge, the present invention has been finally accomplished.

Specifically, the present invention provides a dental-caries detecting solution obtainable by dissolving a dye capable of staining a carious region of a tooth to provide enhanced visibility of the carious region, in a mixture of a water-miscible organic solvent and water. In this dental-caries detecting solution, the water-miscible organic solvent comprises a compound which has a carbon number of 11 or more and includes one or more hydroxyl groups. Further, the water-miscible organic solvent is adjusted such that the dental-caries detecting solution has a viscosity of 300 to 1500 Cp.

In the dental-caries detecting solution of the present invention, the mono-, di- or tri-hydroxy compound having a carbon number of 11 or more, preferably 11 to 50, more preferably 11 to 30 or most preferably 11 to 20, to be used as a water-miscible solvent can dissolve a dye and can be miscible in water at any ratio to form a homogenous solution, as with the conventional dental-caries detecting solutions. As another requirement, the dental-caries detecting solution has to prevent staining caused by the dye from extending to the second layer which should not be removed. As long as the water-miscible organic solvent in the finished dental-caries detecting solution has a viscosity of 300 to 1500 Cp, preferably 500 to 800 Cp, to meet the above requirement, it is not limited to a specific type.

For example, polyethylene glycol, polypropylene glycol, polybutylene glycol or polyglycerin may be suitably used as the water-miscible organic solvent, because a polymerization degree thereof can be appropriately selected to readily adjust its viscosity at a value suitable for the present invention in the same composition. Among them, polyethylene glycol or polypropylene glycol, which has an average molecular weight of 300 to 400, is particularly preferable. Further, a water-miscible organic diluent solvent having a lower viscosity to serve as a diluent, such as water, ethanol, ethylene glycol or propylene glycol may be mixed in the water-miscible organic solvent to adjust the viscosity. The mixing ratio between them may be appropriately determined depending on the type of selected solvent.

The die to be used in the present invention may be any suitable type capable of being dissolved in the above solvent, staining a carious region of a tooth to provide enhanced visibility of the carious region, and preventing the obtained stain from being removed when the stained region is rinsed with water. The dye may have any suitable color discriminable from a tooth color, and a red edible dye is particularly preferable. For example, the dye may be one or more selected from the group consisting of amaranth, erythrosine, Allura red AC, new coccin, phloxine, rose bengal, acid red, eosin, acid fuchsin, basic fuchsin, safranine and rhodamine B. Preferably, the dye is contained in the solution in an amount of 0.1 to 2 mass %. If the content of the dye is less than 0.1 mass %, a carious region cannot be adequately stained in short period of time. If the content of the dye is greater than 2 mass %, the staining will undesirably extend to the second layer which should not be removed, to cause a difficulty in discriminating between the first and second layers and a major production difficulty in dissolving the dye.

The preparation of the composition of the dental-caries detecting solution of the present invention is not limited to a specific process. For example, the process may comprise adding a necessary amount of dye to a selected solvent, and stirring the mixture at room temperature or an appropriately increased temperature to dissolve the die in the solvent, or may comprise preparing a composition containing a dye at a higher concentration than a necessary value in the same manner, and diluting this composition.

In an operation for applying the dental-caries detecting solution of the present invention to a carious region of a tooth, this detecting solution is supplied into a container having an elongated nozzle attached thereto, and a small amount of the detecting solution is dropped directly onto the carious region of the tooth. Then, within a short time of 1 to 10 seconds, the region having the dropped detecting solution is rinsed with water.

According to the dental-caries detecting solution of the present invention, among a carious region of a tooth, only a first decalcified layer having bacterial infection can be stained, absolutely without staining a second decalcified layer having no bacterial infection and a healthy dentine to be left. Thus, only a specific region of the tooth to be removed can be stained in a clearly discriminable manner.

The present invention will now be specifically described in connection with an example.

### [EXAMPLE]

Table 1 shows a mixing ratio in each Inventive Example. Table 2 shows a mixing ratio in each Comparative Example. In Table 2, each of Comparative Examples 1 to 10 has either one of mixing ratios disclosed in the aforementioned Patent Publication 3, and each of Comparative Examples 11 to 14 has either one of mixing ratios disclosed in the aforementioned Patent Publication 4. Comparative Examples 15, 16 and 17 have mixing ratios disclosed in the aforementioned Patent Publications 5, 6 and 7, respectively. Each of Comparative Examples 18 to 20 has a viscosity outside the range of the present invention.

Constituents were mixed at the mixing ratio as shown in Tables 1 and 2. Then, the mixture was stirred while being heated up to 60°C in Inventive Examples 1 to 6 and Comparative Example 20, or at room temperature in the remaining Examples, to prepare various detecting solutions.

As to each of the prepared detecting solution, the viscosity thereof was measured, and the distinctiveness of staining was evaluated. Further, the observation of bacteria was performed using a Gram-stained thin slice segment.

### (Viscosity Measurement)

The viscosity measurement was performed according to the capillary tube viscometer method as the 1st method of Viscosity Test Procedures in the 14-th revision of General Test Procedures defined by Japanese Pharmacopoeia. The result is shown in Table 3.

**Table 1**

| | Dye (weight part) | | Solvent (weight part) | |
|---|---|---|---|---|
| Inventive Example 1 | acid red | 1 | polypropylene glycol (molar weight: 300) | 97 |
| | | | water | 2 |
| Inventive Example 2 | acid red | 0.8 | polyethylene glycol (molar weight: 400) | 89.2 |
| | | | polyethylene glycol (molar weight: 4000) | 5 |
| | | | water | 5 |
| Inventive Example 3 | amaranth | 0.8 | polyethylene glycol (molar weight: 400) | 84.8 |
| | | | polyethylene glycol (molar weight: 4000) | 9.4 |
| | | | water | 7.2 |
| Inventive Example 4 | acid red | 0.8 | polypropylene glycol (molar weight: 300) | 87.2 |
| | | | propylene glycol | 7 |
| | | | water | 5 |
| Inventive Example 5 | phloxine | 0.5 | polypropylene glycol (molar weight: 300) | 43 |
| | | | glycerin | 42.5 |
| | | | propylene glycol water | 9 5 |
| Inventive Example 6 | phloxine | 0.5 | polypropylene glycol (molar weight: 300) | 85 |
| | | | polyethylene glycol (molar weight: 1500) | 9.75 |
| | | | water | 4.75 |

**Table 2**

| | Dye (weight part) | | Solvent (weight part) | | Other Constituent (weight part) | |
|---|---|---|---|---|---|---|
| Comparative Example 1 | Phloxine BK | 1 | propylene glycol | 99 | - | - |
| Comparative Example 2 | acid red | 1 | propylene glycol | 99 | - | - |
| Comparative Example 3 | fast acid magenta | 1 | propylene glycol | 99 | - | - |
| Comparative Example 4 | phloxine B | 1 | propylene glycol | 99 | - | - |
| Comparative Example 5 | fast green FCF | 1 | propylene glycol | 99 | - | - |
| Comparative Example 6 | rhodamine B | 1 | propylene glycol | 99 | - | - |
| Comparative Example 7 | rhodamine B | 1 | triethylene glycol | 99 | - | - |
| Comparative Example 8 | rhodamine B | 2 | tnethylene glycol | 98 | - | - |
| Comparative Example 9 | acid red | 1 | triethylene glycol | 99 | - | - |
| Comparative Example 10 | acid red | 2 | triethylene glycol | 98 | - | - |
| Comparative Example 11 | basic fuchsin | 0.5 | propylene glycol | 100 | - | - |
| Comparative Example 12 | basic fuchsin | 0.5 | propylene glycol | 50 | - | - |
| | | | water | 50 | | |
| Comparative Example 13 | basic fuchsin | 0.5 | propylene glycol | 20 | - | - |
| | | | water | 80 | | |
| Comparative Example 14 | basic fuchsin | 0.5 | ethanol | 95 | - | - |
| | | | water | 5 | | |
| Comparative Example 15 | acid red | 0.5 | propylene glycol | 100 | triclosan | 0.5 |
| Comparative Example 16 | acid red | 0.5 | propylene glycol | 100 | methacryloyl oxy dodecyl pyridinium bromide | 0.5 |
| Comparative Example 17 | acid red | 0.5 | propylene glycol | 100 | methacryloyl oxy butyl pyridinium bromide | 0.5 |
| Comparative Example 18 | acid red | 1 | dipropylene glycol | 99 | - | - |
| Comparative Example 19 | acid red | 1 | tripropylene glycol | 99 | - | - |
| Comparative Example 20 | amaranth | 0.8 | polyethylene glycol (molar weight: 400) | 84.8 | - | - |
| | | | polyethylene glycol (molar weight: 4000) | 9.4 | | |
| | | | water | 5 | | |

**Table 3**

| | Viscosity (Cp) | Staining Distinctiveness (DIAGNOdent measurement value) | | Observation Result of Bacteria |
|---|---|---|---|---|
| Inventive Example 1 | 600 | adequate staining | (12) | no bacteria |
| Inventive Example 2 | 550 | adequate staining | (11) | no bacteria |
| Inventive Example 3 | 630 | adequate staining | (11) | no bacteria |
| Inventive Example 4 | 310 | adequate staining | (8) | no bacteria |
| Inventive Example 5 | 380 | adequate staining | (12) | no bacteria |
| Inventive Example 6 | 1500 | adequate staining | (15) | no bacteria |
| Comparative Example 1 | 50 | excessive staining extending to second layer | (3) | no bacteria |
| Comparative Example 2 | 50 | excessive staining extending to second layer | (4) | no bacteria |
| Comparative Example 3 | 50 | excessive staining extending to second layer | (3) | no bacteria |
| Comparative Example 4 | 50 | excessive staining extending to second layer | (3) | no bacteria |
| Comparative Example 5 | 50 | excessive staining extending to second layer | (3) | no bacteria |
| Comparative Example 6 | 52 | excessive staining extending to second layer | (2) | no bacteria |
| Comparative Example 7 | 38 | excessive staining extending to second layer | (3) | no bacteria |
| Comparative Example 8 | 42 | excessive staining extending to second layer | (3) | no bacteria |
| Comparative Example 9 | 40 | excessive staining extending to second layer | (4) | no bacteria |
| Comparative Example 10 | 40 | excessive staining extending to second layer | (3) | no bacteria |
| Comparative Example 11 | 40 | excessive staining extending to second layer | (4) | no bacteria |
| Comparative Example 12 | 6 | excessive staining extending to second layer | (4) | no bacteria |
| Comparative Example 13 | 2 | excessive staining extending to second layer | (3) | no bacteria |
| Comparative Example 14 | 2 | excessive staining extending to second layer | (4) | no bacteria |
| Comparative Example 15 | 50 | excessive staining extending to second layer | (4) | no bacteria |
| Comparative Example 16 | 50 | excessive staining extending to second layer | (4) | no bacteria |
| Comparative Example 17 | 50 | excessive staining extending to second layer | (4) | no bacteria |
| Comparative Example 18 | 93 | excessive staining extending to second layer | (5) | no bacteria |
| Comparative Example 19 | 60 | excessive staining extending to second layer | (5) | no bacteria |
| Comparative Example 20 | 3000 | insufficient staining | (46) | existence of bacteria |

### (Evaluation of Staining Distinctiveness)

A carious cavity of an extracted human tooth having occlusal fissure caries was opened, and each of the detecting solutions was applied thereto. Then, a resulting stained tooth substance was fully removed using a spoon excavator or a spherical steel bar attached to an electric engine in accordance the staining thereof serving as a marker. After confirming that the floor of the cavity was not stained with the detecting solution, the tooth substance of the cavity floor was diagnosed using a DIAGNOdent® (manufactured by KaVo Dental GmbH.). The DIAGNOdent® is a caries diagnostic apparatus using laser, designed to emit a red laser beam having a single short wavelength of 655 nm from a probe onto a surface of a tooth, and detect a raman spectrum of scattered light reflected by the tooth surface to diagnose caries by utilizing the phenomenon that a carious tooth has a greater raman spectral intensity than that of a healthy tooth.

A DIAGNOdent® measurement value determining the existent of caries is described in "Forefront of Dental Laser - clinical applications to various affections" (Dental Diamond Co. Tokyo), p. 32-33, 1999, "J. Kinoshita, The Nippon Dental Review, No.724", p.97-102, 2003, "A. Senda et al., The Nippon Dental Review, No.691", p.80-84, 2000, and "A. Lussi et al., Eur. J. Oral Sci., 109, 9.14-19, 2001." In these articles, reference values are determined based on 1,900 clinical cases. Specifically, a tooth region having a measurement value of 20 or more is classified into caries to be cut away or removed, and a tooth region having a measurement value of greater than 5 to less than 20 is classified into a decalcified region capable of being re-calcified or which should not be removed. Further, a tooth region having a measurement value of 5 or less is classified into a healthy dentine.

Thus, in this evaluation, a carious region was removed in accordance with staining serving as a marker. Then, after confirming that a cavity floor was not stained with the detecting solution, the above measurement was performed according to a criterion on the staining property of the dental-caries detecting solution that a DIAGNOdent® measurement value of 20 or more in the tooth substance of the cavity floor means insufficient staining, the measurement value of 5 or less means excessive staining extending to the second layer, and the measurement value of greater 5 to less than 20 means adequate staining. The result is shown in Table 3.

### (Observation of Bacteria using Gram-Stained Thin Slice Segment)

In the same manner as that in the above evaluation of staining distinctiveness, each of the detecting solutions was applied to a carious tooth. Immediately after a strained tooth substance was fully removed, the tooth is decalcified using ethylenediamine tetraacetic acid (EDTA). Then, the decalcified tooth was dehydrated and embedded in paraffin to prepare a thin slice segment cut along a plane parallel to the axis of the tooth and passing through the deepest portion of the carious cavity. The obtained thin slice segment was Gram-stained, and it was checked under a microscope whether bacteria exist therein. The observation result is shown in Table 3.

As the result of the observation, after the removal of tooth substance based on staining serving as a marker, the cavity floor in each of Inventive Examples had a DIAGNOdent® measurement value (of greater 5 to less than 20) corresponding to a decalcified region capable of being re-calcified, and no existence of bacteria was observed therein. That is, a tooth substance to be left exists in the cavity floor. This proves that the detecting solution of the present invention has an adequate staining property.

In contrast, after the removal of tooth substance based on staining serving as a marker, the cavity floor in each of Comparative Examples 1 to 19 had a DIAGNOdent® measurement value of 0 to 5 corresponding to a healthy dentine. That is, the detection solution in each of Comparative Examples 1 to 19 has an excessive staining property to a tooth substance, and the staining extends to a region capable of being re-calcified. If a tooth substance is removed using as a marker only the staining with these detecting solutions, it will be excessively removed. No existence of bacteria was observed in the cavity floor after the excessive removal extending to a healthy dentine.

In Comparative Example 20, after the removal of tooth substance based on staining serving as a marker, the cavity floor had a DIAGNOdent® measurement value of 20 or more corresponding to a carious region to be removed, and the existence of bacteria was observed therein. That is, this detecting solution has an insufficient staining property to a tooth substance due to its excessively high viscosity. If a tooth substance is removed using as a marker only the staining with this detecting solution, a carious region to be essentially removed will be partially left.

The present invention provides an effective means capable of informing any dentist about a criterion for a carious region to be removed, with high repeatability.

## Claims

1. A dental-caries detecting solution obtainable by dissolving a dye capable of staining a carious region of a tooth to provide enhanced visibility of said carious region, in a water-miscible organic solvent, wherein said water-miscible organic solvent comprises
a compound which has a carbon number of 11 or more and includes one or more hydroxyl groups, wherein said dental-caries detecting solution has a viscosity of 300 to 1500 Cp.

2. The dental-caries detecting solution as defined in claim 1, wherein said water-miscible organic solvent is polyethylene glycol or polypropylene glycol, which has an average molecular weight of 250 to 400.

3. The dental-caries detecting solution as defined in claim 1, wherein said water-miscible organic solvent is mixed with a water-miscible organic diluent solvent having a lower viscosity to adjust said viscosity.

4. The dental-caries detecting solution as defined in claim 1, wherein said dye is contained in an amount of 0.1 to 2 mass %.

5. Use of a solution as defined in any one of claims 1 to 4 for the preparation of a dental-caries detecting agent.
